# EUROPEAN PATENT APPLICATION

(11) **EP 1 262 487 A1**
(43) Date of publication of application: **04.12.2002**
(21) Application number: 01908221.3
(22) Date of filing: 01.03.2001
(51) Int. Cl.: C07K 14/47, C12N 15/09, C07K 16/18, C12N 5/10, C12Q 1/02

(54) **DNA ENCODING SQUALENE EPOXIDASE PROMOTER**

(30) Priority: 02.03.2000 JP 2000057743
(71) Applicant: CHUGAI SEIYAKU KABUSHIKI KAISHA, Tokyo, 115-8543 (JP)
(72) Inventor: INOUE, Keizo, Hanno-shi, Saitama 357-0041 (JP); ARAI, Hiroyuki, Bunkyo-ku, Tokyo 112-0002 (JP); ARITA, Makoto, Brookline, MA 02446 (US)
(74) Representative: Maschio, Antonio
(86) International application number: JP0101592
(87) International publication number: WO01064740

(57) **Abstract**

Provided is DNA encoding a squalene epoxidase-promoting factor having a function of controlling the activity of squalene epoxidase, which is the rate-limiting enzyme in the cholesterol biosynthesis.

## Description

### TECHNICAL FIELD

The present invention relates to a squalene epoxidase-promoting factor (hereinafter, referred to as "SPF") and a method for its production. The present invention further relates to DNA encoding the factor and the antisense polynucleotide thereof. The present invention still further relates to an antibody against the factor as an antigen, a method for screening a substance inhibiting the factor, and an agent for inhibiting the biosynthesis of cholesterol which comprises a substance inhibiting the factor.

### BACKGROUND ART

A living body provided with a fine regulation mechanism controls cholesterol levels as necessary. It is known that many diseases including arteriosclerosis may develop when such a homeostasis maintenance mechanism fails. In particular, cholesterol synthesis and catabolism in the liver are important elements for regulating cholesterol levels which circulate within the body. To date, many enzymes including HMG-CoA reductase and squalene epoxidase have been identified to be involved in cholesterol biosynthesis. Further, the inhibitors for such enzymes have been recognized as having an improvement effect on hyperlipidemia. Furthermore, it has been reported that a rat liver soluble fraction has an activity promoting an enzyme reaction which converts squalene to squalene 2,3-oxide mediated by squalene epoxidase present in a rat liver microsome (Tchen T. Bloch K. (1957) J. Biol. Chem 226, 921-930). A protein having this activity is referred to as a supernatant protein factor (SPF), and the purification of the protein has also been reported (Ferguson J. Bloch K. (1977) J. Biol. Chem 252, 5381-5385). However, the primary structure and cDNA of the protein remains unknown.

Squalene epoxidase is a rate-limiting enzyme for cholesterol biosynthesis. Hence, if DNA encoding a protein capable of controlling the enzyme activity can be isolated, cholesterol synthesis can be easily controlled in vivo. Further, if SPF can be produced from such DNA by gene engineering techniques, screening for the inhibitor thereof can be facilitated. Since SPF is not directly involved in cholesterol biosynthesis, the inhibitor for the protein can be used as a cholesterol-reducing agent having a site of action different from that of conventional agents.

The object of the present invention is to provide DNA encoding such highly efficient SPF.

### DISCLOSURE OF THE INVENTION

We have performed the following procedures to solve the above problems.
1) Rat liver soluble fractions were purified based on squalene epoxidase-promoting activity as an index.
2) A gene having homology to partial amino acid sequences of the purified protein was found from a human genome DNA sequence database, and primers were designed based on the sequences.
3) Using the designed primers, polymerase chain reactions were performed for rat and human genes.

We have completed the present invention by succeeding for the first time in isolating DNA encoding SPF from rats and humans by performing the above procedures.

First, the present invention relates to DNA encoding SPF.

Second, the present invention relates to SPF.

Third, the present invention relates to an antibody which binds to SPF.

Fourth, the present invention relates to an antisense polynucleotide of DNA encoding SPF.

Fifth, the present invention relates to a method for screening an inhibitory substance for SPF.

Sixth, the present invention relates to a cholesterol biosynthesis inhibitor which comprises the inhibitory substance for SPF.

The present invention includes part or all of the contents disclosed in the specification and drawings of Japanese Patent Application No. 2000-57743, which is a priority document of the present application.

### BRIEF DESCRIPTION OF DRAWINGS

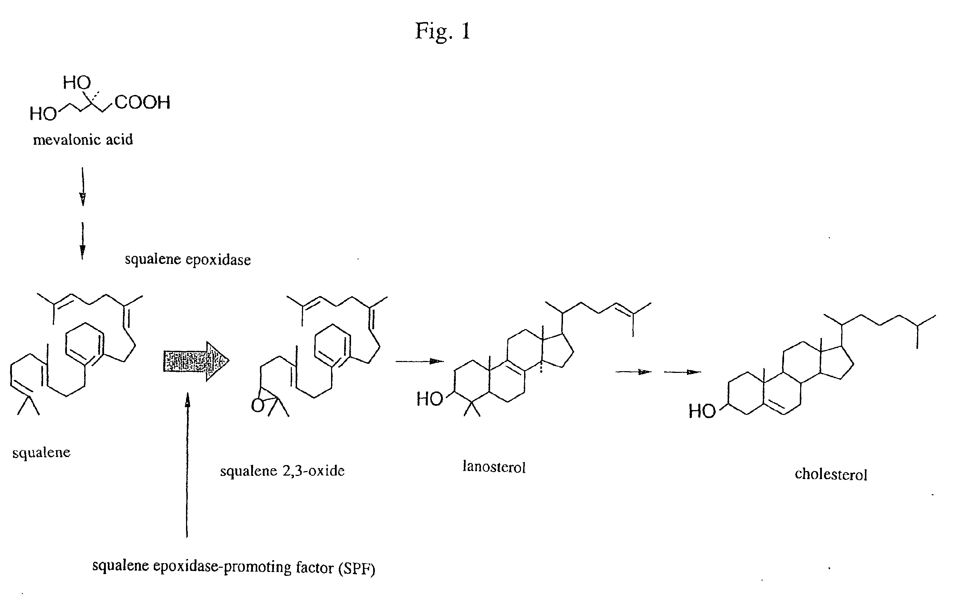

Figure 1 shows a cholesterol biosynthesis pathway.
Figure 2 shows amino acid sequences of SPFs derived from rats and humans.
Figure 3 shows amino acid sequences of proteins having high homology to human-derived SPFs.
Figure 4 shows the amounts of squalene and squalene 2,3-oxide synthesized in cells to which DNA encoding rat-derived SPF was introduced.
Figure 5 shows the intermembrane squalene-transporting activity of rat-derived SPF.
Figure 6 shows the amount of SPF expressed in a rat and a human.

### BEST MODE FOR CARRYING OUT THE INVENTION

The present invention will be described in detail for each invention.

### (1) First invention (DNA)

The DNAs of the present invention include DNA encoding an amino acid sequence of SEQ ID NO: 2 and DNA encoding an amino acid sequence of SEQ ID NO: 4, and DNA represented by SEQ ID NO: 1 and DNA represented by SEQ ID NO: 3.

In addition, persons skilled in the art can easily produce by standard techniques a protein functionally equivalent to the protein having an amino acid sequence represented by SEQ ID NO: 2 or 4 by, for example, appropriate substitution of an amino acid(s) so as not to affect the functions. As such, DNAs of the invention also encompass DNA encoding a protein represented by an amino acid sequence having deletion, substitution or addition of one or more amino acids in the amino acid sequence of SEQ ID NO: 2 or 4, and having squalene epoxidase-promoting activity. Examples of standard techniques include a site-directed mutagenesis system with PCR (GIBCO-BRL, Gaithersburg, Maryland) and a site-directed mutagenesis method with oligonucleotides (Kramer, W. and Fritz, HJ (1987) Methods in Enzymol., 154:350-367). The number of amino acids to be altered is normally 30 amino acids or less, preferably 20 amino acids or less, more preferably 10 amino acids or less, and still further preferably 5 amino acids or less.

It is also a standard practice for persons skilled in the art to isolate DNA having high homology with and functionally equivalent to the nucleotide sequence (or a part of the sequence) of SEQ ID NO: 1 or 3 by a well known hybridization technique (Sambrook, J et al. Molecular Cloning 2nd ed. 9.47-9.58, Cold Spring Harbor Lab. press, 1989). Thus, the DNA of the invention also encompasses DNA which is complementary to DNA hybridizing and functionally equivalent to the DNA represented by the nucleotide sequence of SEQ ID NO: 1 or 3. Stringent conditions employed herein for hybridization normally indicate about "42C°, 2xSSC, 0.1%SDS," preferably about "50C°, 2xSSC, 0.1% SDS," and more preferably about "65C°, 2xSSC, 0.1%SDS." DNA obtained using hybridization techniques normally shares high homology in the nucleotide sequence with the DNA represented by the nucleotide sequence of SEQ ID NO: 1 or 3. The term "high homology" means 60% or more homology, preferably 75% or more homology, and more preferably 90% or more homology.

The DNAs of the present invention include cDNA, genomic DNA, chemically synthesized DNA and the like. For example, cDNA can be prepared by preparing primers based on the nucleotide sequence of SEQ ID NO: 1 or 3, and performing RT-PCR. Further, genomic DNA can be prepared by a plaque hybridization method using λ phages. The nucleotide sequence of the obtained DNA can be determined by standard techniques using, for example, a commercially available "dye terminator sequencing kit" (Applied Biosystems).

### (2) Second invention (protein)

The proteins of the present invention include both a recombinant protein which is prepared by gene recombination techniques and an isolated natural protein.

The recombinant protein can be prepared by, for example, a method which involves incorporating the DNA of the present invention (for example, DNA having the nucleotide sequence of SEQ ID NO: 1 or 3) into an appropriate expression vector, introducing the vector into a host cell to produce a transformant, and purifying the protein from the transformant. An expression vector preferably used herein is, for example, "pQE vector" (Qiagen, Hilden, Germany) when *E.coli* is used, "SP-Q01" (Stratagene, La Jolla, California) when yeast is used, and "BAC-to-BAC baculovirus expression system" (GIBCO-BRL, Gaithersburg, Maryland) when insect cells are used. Further, the DNA of the invention can be incorporated into a vector by standard techniques (for example, see "The Qiaexpressionnist handbook, Qiagen, Hilden, Germany"). Any host cell can be used, so far as the cell can produce the protein of the invention. Examples of such a host cell that can be used herein include *E. coli,* yeast, animal cells, and insect cells. Vectors can be introduced into cells, for example, by electroporation or a calcium phosphate method. In addition, isolation and purification of recombinant proteins from transformants can be performed by a standard method, such as a method described in literature "The Qiaexpressionnist handbook, Qiagen, Hilden, Germany."

Natural protein can be isolated by, for example, preparing a column onto which antibodies binding to the protein of the present invention have been immobilized, and performing affinity chromatography using the column for tissues or cell extracts with high expression of the protein of the invention. Examples of tissues or cells highly expressing the protein of the invention include liver, brain, small intestine, prostate glands, kidney, pancreas and peripheral leucocytes.

### (3) Third invention (antibody)

The antibody of the present invention may be in any form, so far as it binds to the protein of the invention. Examples of the antibody include both polyclonal and monoclonal antibodies. In addition, examples of the antibody of the invention include antiserum produced by immunization of a rabbit or the like with the protein of the invention, polyclonal and monoclonal antibodies of all the classes, humanized antibodies produced by gene recombination, and human antibodies.

The antibody of the present invention can be prepared by the following methods. For example, polyclonal antibodies can be prepared by immunizing small animals, such as rabbits, with the protein of the invention to obtain serum; applying the serum to the affinity column with the protein of the invention immobilized thereon to obtain fractions which recognize only the protein of the invention; and purifying, from the obtained fractions, immunoglobulin G or M with a protein A or G column. Further, monoclonal antibodies can be prepared by a method which involves immunizing a small animal, such as a mouse, with the protein of the invention; excising the spleen from the mouse; crushing the spleen to prepare cells; allowing the cells to fuse to mouse myeloma cells with a reagent, such as polyethylene glycol; selecting clones which produce antibodies for the protein of the invention from the resulting fused cells (hybridomas); transplanting the hybridomas to a mouse intraperitoneally; collecting the ascites of the mouse; and purifying the resulting monoclonal antibodies by, for example, ammonium sulfate precipitation, protein A column, protein G column, DEAE ion exchange chromatography, the affinity column with the protein of the invention immobilized thereto or the like.

The antibody of the present invention can be used as an agent for promoting or inhibiting the function of the protein of the invention (specifically, squalene epoxidase-promoting activity), in addition to being used for purifying the protein of the invention. When the antibody is administered as an agent to a human body, a human antibody or a humanized antibody is effective in terms of immunogenicity. Human antibodies can be prepared by immunizing a mouse, whose immune system has been replaced with a human immune system, with the protein of the invention. Further, humanized antibodies can be prepared by, for example, a CDRgrft method which involves cloning antibody genes from monoclonal antibody-producing cells, and grafting the antigenic determinant sites to human antibodies.

### (4) Fourth invention (antisense polynucleotide)

The polynucleotide of the present invention comprises a nucleotide sequence which is complementary to the whole or a part of the nucleotide sequence of the DNA of the invention or of mRNA synthesized from the DNA.

The polynucleotide of the present invention mainly indicates a substance comprising multiple linkage of a nucleotide consisting of a base, a phosphate group, and a sugar. The polynucleotide of the invention also includes a polynucleotide analog which is analogous to a polynucleotide in the three-dimensional structures and functions. Examples of such a polynucleotide analog include an analog in which a substance is added to the 3' or 5' terminus of a polynucleotide; an analog in which part of bases, sugars and/or phosphate groups is modified by, for example, substitution, deletion or addition; an analog which comprises artificial base, sugar and/or phosphate group which do not exist in nature; or an analog which has a backbone other than the sugar-phosphate backbone.

The polynucleotide of the present invention may hybridize to any part of the DNA of the present invention.

The polynucleotide of the present invention can be used as a probe for examining the presence and the expression of the DNA or the protein of the invention in tissues or cells. When the polynucleotide is used as a probe, preferably the number of bases is 12 or more and GC content% is 30 to 70%, and particularly preferably the number of bases is 16 or more and the GC content% is 30 to 70%.

The polynucleotide of the present invention can be used for regulating expression of the protein of the invention. The protein of the present invention promotes a epoxidation reaction of squalene in the cholesterol biosynthesis pathway. Hence, regulating expression of the protein enables regulation of cholesterol biosynthesis.

In general, a method for regulating expression of a polypeptide using a polynucleotide comprising a nucleotide sequence complementary to mRNA or DNA encoding the polypeptide is referred to as an antisense method. It is thought that the polynucleotide having a complementary sequence binds to DNA or mRNA carrying genetic information at any one of stages of: 1) transcription from a gene to a precursor mRNA, 2) processing from the precursor mRNA to a mature mRNA, 3) passing across the nuclear membrane and 4) translation into a protein, so as to affect a normal flow of genetic information, thereby regulating expression of the polypeptide. It is generally thought that a nucleotide sequence comprising 15 or more nucleotides has specificity (Kazunari YOKOYAMA, Protein, Nucleic Acid, Enzyme, Vol. 38, p754 to 765, 1994). Accordingly, it is assumed that the polynucleotide of the invention comprising a nucleotide sequence of 15 or more nucleotides specifically binds to DNA of the invention or mRNA synthesized therefrom. In contrast, when the sequence of a polynucleotide is too long, it cannot be incorporated into a cell. Therefore, when the polynucleotide of the invention is used for regulating expression of protein, the polynucleotide of the invention has a complementary sequence which comprises 15 or more and 30 or less nucleotides, preferably, 15 or more and 25 or less nucleotides, more preferably, 18 or more and 22 or less nucleotides.

The polynucleotide of the present invention can be altered in many ways for improving the effect thereof using known antisense techniques. Such alterations include a procedure for improving its ability to bind with a target DNA or mRNA, tissue selectivity, cell-permeability, nuclease resistance, intracellular stability or the like.

To facilitate hybridization, a polynucleotide is preferably designed to have a nucleotide sequence complementary to the nucleotide sequence of a region forming a stem-loop ("Clinical Immunology, Vol. 25," p1200-1206, 1993). The polynucleotide of the invention may be designed so as to form a stem-loop, if necessary.

In general, it can be assumed that a polynucleotide with a sequence complementary to the sequence of a region in the vicinity of a translation initiation codon, a ribosome binding site, a capping site or a splice site has a high inhibitory effect on expression ("Cancer and Chemotherapy, vol. 20, No. 13," p1899-1907). Thus, the polynucleotide of the present invention can also be designed to comprise such sequences for improving an inhibitory effect on expression.

As described above, examples of the polynucleotide of the present invention that can be used herein include polynucleotide analogs having backbones other than sugar-phosphate backbones. A preferable example among such analogues is a substance having a phosphorothioate bond as a backbone structure (see "Cancer and Chemotherapy, Vol. 20, No. 13," p1899-1907, 1993).

An example of a method for producing the polynucleotide of the present invention is described in "in Antisense Research and Applications" (Michael J. GAIT, p290-299, CRC, Florida, 1993). For example, when native DNA or RNA is used, the polynucleotide of the invention can be produced by synthesizing with a chemical synthesizer, or by a PCR method using the DNA of the invention as a template. In addition, some polynucleotide analogs, such as methylphosphonate-type or phosphorothioate-type analogs, can be synthesized with a chemical synthesizer (for example, type 394, Perkin Elmer Japan). In this case, the target polynucleotide can be produced by performing procedures as described in the instruction attached to a chemical synthesizer, and purifying the synthesized products by an HPLC method using reverse phase chromatography or the like.

### (5) Fifth invention (Screening method)

A method for screening an inhibitory substance for the squalene epoxidase-promoting factor of the present invention comprises allowing co-existence of the protein of the invention and a test sample, and selecting substances inhibiting squalene epoxidase-promoting activity of the protein of the invention from the test sample; or inoculating a test sample into a cell expressing the DNA of the invention, and selecting substances inhibiting expression of the DNA of the invention from the test sample.

Examples of a test sample to be screened include, but are not limited to, cell extracts, libraries of synthetic low molecular weight compounds, expression products of gene libraries and libraries of synthetic peptides.

Cells expressing the DNA of the present invention can be prepared by introducing a vector containing the DNA of the invention into an appropriate host cell. A test sample can be inoculated into a cell by standard techniques according to the type of the test sample. Whether the test sample inhibits expression of the DNA of the invention can be determined, for example, by disrupting cells after inoculation, and measuring the amount of the protein of the invention in the solution containing the disrupted cells.

The thus selected inhibitory substances may be applied as various agents. When these inhibitory substances are used as agents, they may be formulated by any known pharmaceutical production method. For example, the agent may be administered to a patient with pharmacologically acceptable carriers or media (e.g., physiological saline, vegetable oil, a suspending agent, a surfactant or a stabilizer). Examples of routes of administration include, depending on the properties of a compound to be administered, percutaneous, intranasal, transbronchial, intramuscular, intravenous and oral administrations. Further, when the compound to be selected is DNA, it can be administered to a human body using a vector to be expressed in vivo, such as an adenovirus vector "pAdexLcw" and a retrovirus vector, "pZIPeno."

### (6) Sixth invention (Cholesterol biosynthesis inhibitor)

The cholesterol biosynthesis inhibitor of the present invention comprises a substance inhibiting squalene epoxidase activity of the protein of the invention, or a substance inhibiting expression of the DNA of the invention.

These inhibitors can be produced by the above-mentioned screening method of the present invention. Examples of substances inhibiting the squalene epoxidase activity of the protein of the invention include, but are not limited to, an antibody binding to the active site of the protein of the invention, and an antagonist of the protein of the invention. An example of the substance inhibiting expression of the DNA of the invention is, but is not limited to, an antisense nucleic acid. These inhibitory substances can be formulated by the above-mentioned, known pharmaceutical production method.

### EXAMPLE

### [Example 1] Isolation of DNA encoding SPFs derived from rats and humans

SPF was purified from rat liver soluble fractions based on squalene epoxidase-promoting activity as an index. For purification, acetone fractionation (40%-75%), Hi Prep Sephacryl S-300 16/60 HR column (Pharmacia), Q-Sepharose column (Pharmacia) Econo-Pac CHT-II (Bio-Rad), and Mono S HR5/5 column (Pharmacia) were used in combination. Finally, SPF could be purified by these procedures so that SPF was detected as a single band on SDS-PAGE. The purified SPF was extracted from acrylamide gel. After limited decomposition, partial amino acid sequences at two positions (SEQ ID NO: 5/ HISPDQLPVEYGGTMTDPD) (SEQ ID NO: 6/ENVQDVLPALPNPDDYFLL) were determined using 477A protein sequencer (Applied Biosystems).

A search through a human genome DNA sequence database (Genbank) for a gene having homology with these partial amino acid sequences revealed that a sequence registered as hypothetical protein (AL096881) shared homology of 90% or more with the partial amino acid sequences.

Based on the sequence information, the following primers were designed to amplify the full length DNAs encoding SPFs derived from a rat and a human.

Primers for amplification of rat-derived SPF
"rSPF-1" (SEQ ID NO : 7/5'-CAGCGCGCCCCAAGGCTGAG-3')
"rSPF-2" (SEQ ID NO : 8/5'-GACTAAAGAAACTGACAGGA-3')

Primers for amplification of human-derived SPF
"hSPF-1" (SEQ ID NO : 9/5'-ATGAGCGGCAGAGTCGGCGA-3')
"hSPF-2" (SEQ ID NO : 10/5'-TTATTTCGGGGTGCCTGCCC-3')

DNA encoding rat-derived SPF was amplified by RT-PCR using RNA purified from a rat liver as a template, "rSPF-1" and "rSPF-2" as primers, and Pyrobest DNA polymerase (Takara Shuzo). The amplified fragments were sub-cloned into pT7Blue (Novagen), thereby obtaining a plasmid "pT7Blue-rSPF."

Further, DNA encoding human-derived SPF was amplified by RT-PCR using Human liver total RNA (Sawady Tech) as a template, "hSPF-1" and "hSPF-2" as primers, and Pyrobest DNA polymerase (Takara Shuzo). The amplified fragments were sub-cloned into pT7Blue (Novagen), thereby obtaining a plasmid "pT7Blue-hSPF."

The nucleotide sequences of the full length DNAs encoding SPFs contained in both clones were determined using ABI PRISM Dye Terminator Cycle Sequencing Ready Reaction Kit, 377 DNA Sequencer(Applied Biosystems). The nucleotide sequence of DNA derived from a rat and that of DNA derived from a human are shown in SEQ ID NOS: 1 and 3, respectively. Further, deduced amino acid sequences encoded by each DNA are respectively shown in SEQ ID NO: 2 and 4.

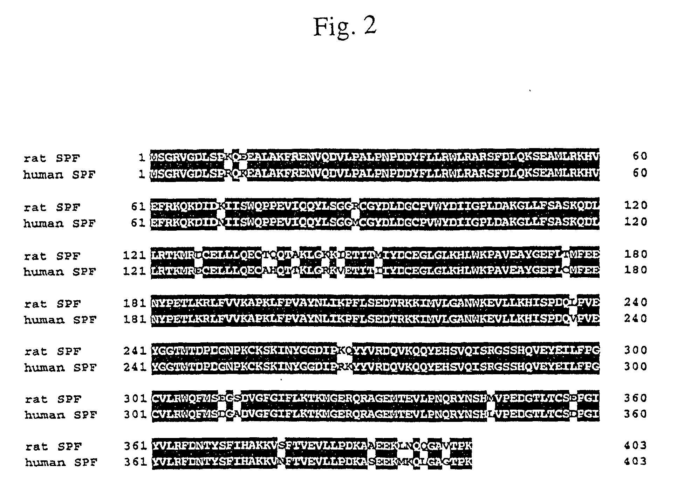

As shown in SEQ ID NOS: 2 and 4, rat-derived SPF consists of 403 amino acid residues and has a putative molecular weight of 46,165; and human-derived SPF consists of 403 amino acid residues and has a putative molecular weight of 46,144. Further, the homology between the amino acid sequences of both proteins was 93.8% (Fig. 2).

A homology search was performed through the existing database (Genbank) for the amino acid sequences of both proteins. Thus, two types of human proteins (AAF19258, AAF19259), and one type of rat protein (AJ132352) with unknown functions were found to show 75% or more homology with the amino acid sequences. These proteins were designated hSLP1, hSLP2 and rSLP1, respectively, since they were SPF-like proteins.

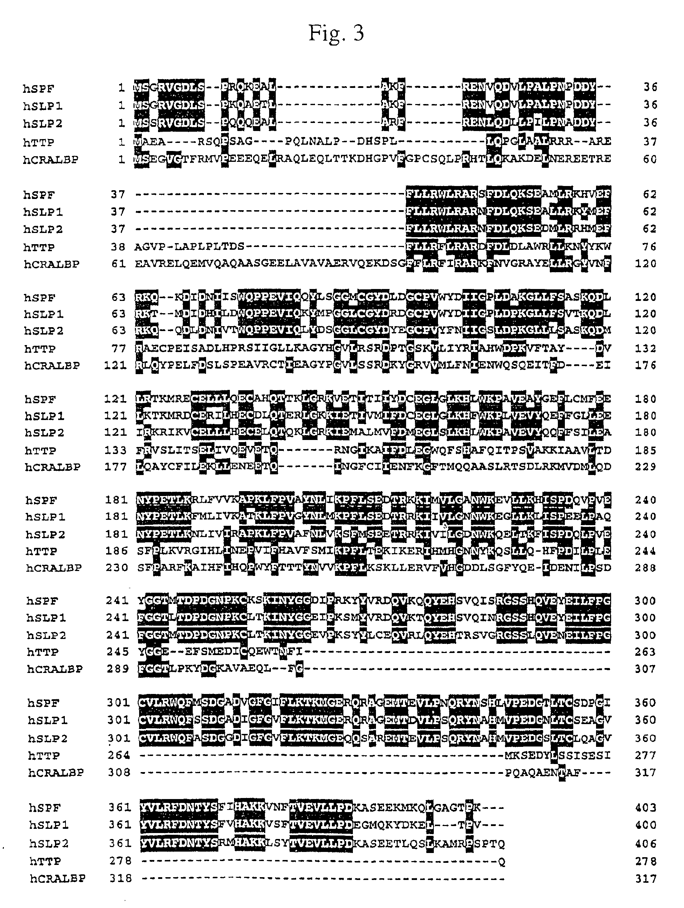

Amino acid sequences were compared among the human-derived SPF, "hSLP1," "hSLP2," "human alpha-tocopherol transfer protein, hTTP (D49488),"and "human 11-cis-retinaldehyde binding protein, hCRALBP (L34219)" (Fig. 3). Based on the similarity in primary structure, the SPF is considered to belong to a lipid transport protein family.

### [Example 2] Measurement of squalene epoxidase-promoting activity of SPF derived from rat

PCR was performed using "pT7Blue-rSPF" as a template, and primers "rSPF-3" (SEQ ID NO: 11/5'-ATGGATCCATGAGCGGCAGAGTCGGTGACCTG-3') and "rSPF-4" (SEQ ID NO: 12/5'-AAGTCGACTTATTTGGGGGTGTCTGC-3'). The amplified fragments were treated with restriction enzymes BamHI and SalI, and then inserted into a plasmid pcDNA3 (Invitrogen), thereby obtaining a plasmid "pcDNA3-rSPF." Similarly, PCR was performed using "pT7Blue-hSPF" as a template and primers "hSPF-3" (SEQ ID NO: 13/5'-CCGGAATTCATGAGCGGCAGAGTCGGCG-3') and "hSPF-4" (SEQ ID NO: 14/5'-CGCGTCGACTTATTTCGGGGTGCCTGC-3'), thereby obtaining a plasmid "pcDNA3-hSPF."

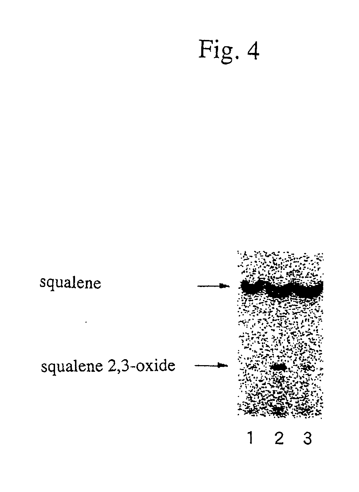

Rat liver cancer-derived cell line McARH7777 (obtained from ATCC) was transfected with "pcDNA3-rSPF" using Superfect Transfection Reagent (Qiagen). Lysates were prepared from the cells, and then squalene epoxidase-promoting activity (SP activity) in the soluble fractions was measured according to a method described in Ferguson J. Bloch K. (1977) J. Biol. Chem 252, 5381-5385. Specifically, tritium-labeled squalene was added to the soluble fractions of the lysate, and then cofactors, such as FAD, NADPH, phosphatidylglycerol or the like, and oxidosqualene cyclase inhibitor Amo-1618 (Calbiochem) were added to the fractions. Following reaction at 37°C for 30 min, lipid components were extracted with an organic solvent, followed by thin-layer chromatography for separation into each component. As a control, the cell line was transfected with "pcDNA3" instead of "pcDNA3-rSPF", and then procedures were performed in the same manner as described above. Figure 4 shows the result. In Fig. 4, lane 1 represents the result obtained with a buffer only, lane 2 represents the result of transfection with "pcDNA3-rSPF," and lane 3 represents the result of transfection with "pcDNA3." As shown in Fig. 4, transfection with "pcDNA3-rSPF" resulted in an increase in the amount of squalene 2,3-oxide synthesized, suggesting that SPF expressed herein resulted in enhanced squalene epoxidase activity.

In addition, similar procedures performed for "pcDNA3-hSPF" yielded the result similar to that of transfection with "pcDNA3-rSPF."

### [Example 3] Measurement of intermembrane squalene-transporting activity of SPF derived from rat

PCR was performed using "pT7Blue-rSPF" as a template, and primers "rSPF-3" (SEQ ID NO: 11/5'-ATGGATCCATGAGCGGCAGAGTCGGTGACCTG-3') and "rSPF-5" (SEQ ID NO: 15/5'-TTCTCGAGTTTGGGGGTGACT-3'). The amplified fragments were treated with restriction enzymes BamHI and XhoI, and then inserted to a plasmid pET-21a (Novagen), thereby obtaining a plasmid "pET-21a-rSPF." Similarly, PCR was performed using "pT7Blue-hSPF" as a template and primers "hSPF-3" (SEQ ID NO: 13/5'-CCGGAATTCATGAGCGGCAGAGTCGGCG-3') and "hSPF-5" (SEQ ID NO: 16/5'-TACTCGAGTTTCGGGGTGCCT-3'), thereby obtaining a plasmid "pET-21a-hSPF."

"pET-21a-rSPF" was introduced into *E. coli* BL21 (Novagen), and then expression of SPF was induced with IPTG. Since T7 tag and His tag were added to recombinant proteins to be synthesized in this system, rat-derived SPF was purified using His-Bind Resin (Novagen),

Intermembrane squalene-transporting activity was measured for the purified rat-derived SPF. The activity was measured as follows.

Liposomes containing tritium-labeled squalene were prepared from egg yolk lecithin. Transportation of squalene from the liposome to a rat liver membrane precipitation fraction (centrifuged at 10,000xg) was measured. Specifically, the liposome and the rat liver precipitation (centrifuged at 10,000xg, 500mg protein) were mixed, and then a SPF sample was added to the mixture, followed by incubation at 37°C for 30 min. The mixture was centrifuged at 10,000xg to isolate precipitation membrane fractions, and then radioactivity of the supernatant was measured with a liquid scintillator. In addition, ¹⁴C-labeled triolein (Amersham) was used as a non-specific transport marker.

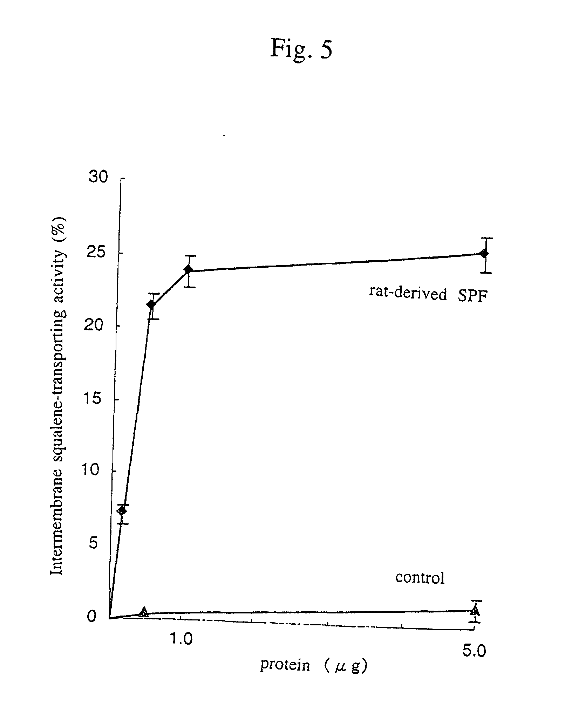

Figure 5 shows the result. As shown in Fig. 5, it was confirmed that the rat-derived SPF had high intermembrane squalene-transporting activity.

Similar procedures performed for "pET-21a-hSPF" yielded a result similar to that for "pET-21a-rSPF."

### [Example 4] Effect of SPF derived from rat on intracellular cholesterol biosynthesis

Rat liver cancer-derived cell line McARH7777 (obtained from ATCC) was transfected with "pcDNA3-rSPF" using Superfect Transfection Reagent (Qiagen). At 48 hours after transfection, addition of [¹⁴C] acetic acid was started, and then 3 hours later radioactivity incorporated in cholesterol was measured. Further, as a control, the cell line was transfected with "pcDNA3" instead of "pcDNA3-rSPF," and then the same procedures as described above were performed. Thus, radioactivity detected from cholesterol in cells transfected with "pcDNA3-rSPF" was about twice as much as that in the control.

Similar procedures performed for "pcDNA3-hSPF" yielded a result similar to that for "pcDNA3-rSPF."

### [Example 5] Analysis of expression of SPFs derived from rat and human in various tissues

Expression of SPFs derived from rats and humans was examined for various tissues by the Northern Blot technique. Approximately 1,200 bp fragments obtained by treating "pcDNA3-rSPF" or "pcDNA3-hSPF" with BamHI-SalI were used as a probe for rat-derived SPF and a probe for human-derived SPF, respectively.

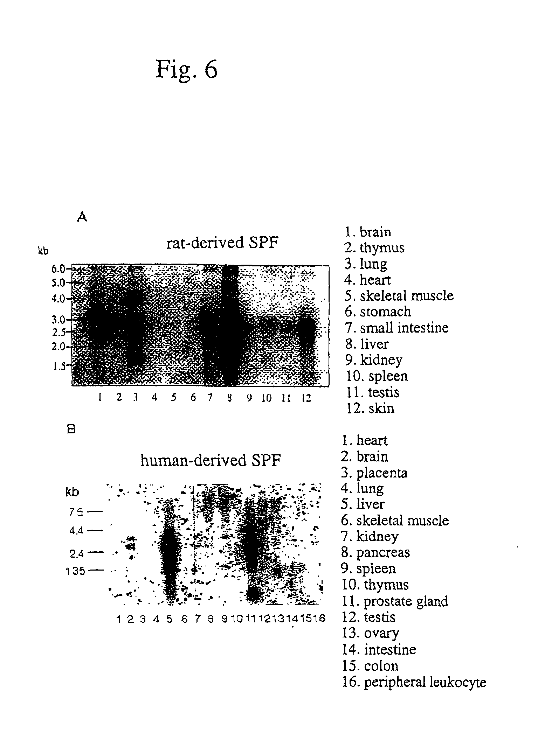

These probes were labeled with [alpha-³²P]dCTP by a random primer method using rediprime II (Amersham pharmacia biotech). Next, the probes were subjected to hybridization with filters of Multiple choice northern blots (Rat) #2, #5 (Sawady Tech) and Multiple tissue northern blots (Human) #1, #2 (Clontech) in Rapid-hyb buffer (Amersham) according to the method recommended by the manufacturer. Figure 6 shows the result. The rat-derived SPF was strongly expressed in the liver, brain, small intestine, lungs and skin (Fig. 6A). On the other hand, the human-derived SPF was strongly expressed in the liver, prostate glands and brain, and weakly expressed in the kidney, pancreas and peripheral leucocytes (Fig. 6B).

All publications, patents and patent applications cited herein are incorporated herein by reference in their entirety.

### INDUSTRIAL APPLICABILITY

The present invention provides DNA encoding SPF. SPF regulates the activity of squalene epoxidase, which is a rate-limiting enzyme in cholesterol biosynthesis. Hence, use of DNA encoding the factor enables facilitated control of in vivo cholesterol biosynthesis. Moreover, realization of producing SPF from the DNA leads to facilitated screening for the inhibitor. Since SPF is not directly involved in cholesterol biosynthesis, the inhibitor for the protein can be used as a cholesterol-reducing agent having a site of action different from that of conventional agents.

## Claims

1. DNA encoding the following protein (a), (b) or (c):
(a) a protein represented by an amino acid sequence of SEQ ID NO: 2;
(b) a protein represented by an amino acid sequence of SEQ ID NO: 4;
(c) a protein represented by an amino acid sequence having deletion, substitution or addition of one or more amino acid in the amino acid sequence of SEQ ID NO: 2 or 4, and having squalene epoxidase-promoting activity.

2. DNA of the following (a), (b) or (c):
(a) DNA represented by a nucleotide sequence of SEQ ID NO: 1;
(b) DNA represented by a nucleotide sequence of SEQ ID NO: 3;
(c) DNA complementary to DNA hybridizing under stringent conditions with the DNA of (a) or (b) and encoding a protein having squalene epoxidase-promoting activity.

3. A protein of the following (a), (b) or (c):
(a) a protein represented by an amino acid sequence of SEQ ID NO: 2;
(b) a protein represented by an amino acid sequence of SEQ ID NO: 4;
(c) a protein represented by an amino acid sequence having deletion, substitution or addition of one or more amino acids in the amino acid sequence of SEQ ID NO: 2 or 4, and having squalene epoxidase-promoting activity.

4. A method for producing the protein of claim 3, which comprises introducing a vector expressing the DNA of claim 1 or 2 into a host cell to produce transformants, and isolating the protein of claim 3 from the transformants.

5. A method for producing the protein of claim 3, which comprises preparing a column with antibodies binding to the protein of claim 3 immobilized thereto, performing affinity chromatography using the column for cell extracts or tissues with high expression of the protein of claim 3, and isolating the protein of claim 3.

6. An antibody which binds to the protein of claim 3.

7. A polynucleotide which comprises a nucleotide sequence complementary to the whole or a part of the nucleotide sequence of the DNA of claim 1 or 2, or of mRNA synthesized from the DNA.

8. A method for screening an inhibitory substance for a squalene epoxidase-promoting factor, which comprises allowing co-existence of the protein of claim 3 and a test sample, and selecting a substance inhibiting squalene epoxidase activity of the protein of claim 3 from the test sample.

9. A method for screening an inhibitory substance for a squalene epoxidase-promoting factor, which comprises inoculating a test sample to a cell expressing the DNA of claim 1 or 2, and selecting a substance inhibiting expression of the DNA of claim 1 or 2 from the test sample.

10. A substance obtained by the method for screening of claim 8 or 9.

11. A cholesterol biosynthesis inhibitor, which comprises a substance inhibiting squalene epoxidase-promoting activity of the protein of claim 3.

12. A cholesterol biosynthesis inhibitor, which comprises a substance inhibiting expression of the DNA of claim 1 or 2.


SEQUENCE LISTING

<110> CHUGAI SEIYAKU KABUSHIKI KAISHA

<120> SQUALENE EPOXIDASE PROMOTING FACTOR GENE

<130> PH-1123-PCT

<150> JP 2000-57743
<151> 2000-03-02

<160> 16

<170> PatentIn Ver. 2.0

<210> 1
<211> 1212
<212> DNA
<213> Rattus norvegicus

<220>
<221> CDS
<222> (1)..(1209)

<400> 1
atg agc ggg aga gtc ggt gac ctg agc ccc aaa cag gag gag gca ctg   48
Met Ser Gly Arg Val Gly Asp Leu Ser Pro Lys Gln Glu Glu Ala Leu
  1               5                  10                  15

gcc aag ttt cga gaa aat gtc cag gac gtg ctg cct gcc ctg ccc aat 96
Ala Lys Phe Arg Glu Asn Val Gln Asp Val Leu Pro Ala Leu Pro Asn
            20                  25                  30

cca gat gac tac ttt ctc ctt cga tgg ctc cga gcc cga agc ttt gac 144
Pro Asp Asp Tyr Phe Leu Leu Arg Trp Leu Arg Ala Arg Ser Phe Asp
        35                  40                  45

ctg cag aag tcg gag gcc atg ctc cgg aag cat gtg gaa ttc cgg aag 192
Leu Gln Lys Ser Glu Ala Met Leu Arg Lys His Val Glu Phe Arg Lys
    50                  55                  60

caa aag gac att gac aaa atc atc agc tgg cag cca cca gag gtg atc 240
Gln Lys Asp Ile Asp Lys Ile Ile Ser Trp Gln Pro Pro Glu Val Ile
65                  70                  75                  80

caa cag tat ctg tca ggc ggc cgg tgt ggg tat gac ctg gac ggc tgc 288
Gln Gln Tyr Leu Ser Gly Gly Arg Cys Gly Tyr Asp Leu Asp Gly Cys
                85                  90                  95

cct gtc tgg tat gac atc atc ggc cca ctg gat gcc aag ggt ctg ctg 336
Pro Val Trp Tyr Asp Ile Ile Gly Pro Leu Asp Ala Lys Gly Leu Leu
            100                 105                 110

ttc tcc gcc tcc aag caa gac ctg ctc agg acc aag atg aga gat tgt 384
Phe Ser Ala Ser Lys Gln Asp Leu Leu Arg Thr Lys Met Arg Asp Cys
        115                 120                 125

gag ctg ctt ctg cag gag tgc acc cag cag acc gca aag cta ggg aag 432

Glu Leu Leu Leu Gln Glu Cys Thr Gln Gln Thr Ala Lys Leu Gly Lys
    130                 135                 140

aag ata gag acc atc acc atg att tac gac tgt gag gga ctc ggc ctc 480
Lys Ile Glu Thr Ile Thr Met Ile Tyr Asp Cys Glu Gly Leu Gly Leu
145                 150                 155                 160

aag cac ctc tgg aaa cct gca gtg gag gcc tat gga gag ttt ctc acc 528
Lys His Leu Trp Lys Pro Ala Val Glu Ala Tyr Gly Glu Phe Leu Thr
                165                 170                 175

atg ttt gag gag aac tat cct gaa acg ctg aag cgg ctg ttc gtc gtt 576
Met Phe Glu Glu Asn Tyr Pro Glu Thr Leu Lys Arg Leu Phe Val Val
            180                 185                 190

aaa gcc ccc aag ctg ttt cca gtg gcc tac aac ctc atc aag ccc ttc 624
Lys Ala Pro Lys Leu Phe Pro Val Ala Tyr Asn Leu Ile Lys Pro Phe
        195                 200                 205

ctg agt gag gac act agg aag aag atc atg gtt ctg gga gca aac tgg 672
Leu Ser Glu Asp Thr Arg Lys Lys Ile Met Val Leu Gly Ala Asn Trp
    210                 215                 220

aag gag gtt tta ctc aaa cac atc agc ccc gac cag ttg cct gtg gag 720
Lys Glu Val Leu Leu Lys His Ile Ser Pro Asp Gln Leu Pro Val Glu
225                 230                 235                 240

tat gga ggc acc atg acg gac cct gat gga aac ccc aag tgt aaa tcc 768
Tyr Gly Gly Thr Met Thr Asp Pro Asp Gly Asn Pro Lys Cys Lys Ser

                245                 250                 255

aag atc aac tat ggg ggt gac atc ccc aag cag tat tac gtg cga gac 816
Lys Ile Asn Tyr Gly Gly Asp Ile Pro Lys Gln Tyr Tyr Val Arg Asp
            260                 265                 270

cag gtg aag cag cag tat gaa cac agt gtg cag atc tcc cga ggc tcc 864
Gln Val Lys Gln Gln Tyr Glu His Ser Val Gln Ile Ser Arg Gly Ser
        275                 280                 285

tcc cac caa gta gag tat gag att ctt ttc ccc ggc tgc gtc ctc agg 912
Ser His Gln Val Glu Tyr Glu Ile Leu Phe Pro Gly Cys Val Leu Arg
    290                 295                 300

tgg cag ttt atg tcg gag gga tca gac gtg ggt ttt ggg att ttc ctg 960
Trp Gln Phe Met Ser Glu Gly Ser Asp Val Gly Phe Gly Ile Phe Leu
305                 310                 315                 320

aag acc aag atg ggg gaa cgg cag cgg gca ggg gag atg acg gag gtg 1008
Lys Thr Lys Met Gly Glu Arg Gln Arg Ala Gly Glu Met Thr Glu Val
                325                 330                 335

ctg cct aac cag aga tac aat tcc cac atg gtc cct gag gat ggg acc 1056
Leu Pro Asn Gln Arg Tyr Asn Ser His Met Val Pro Glu Asp Gly Thr
            340                 345                 350

ctc acc tgc agt gag cca ggc atc tat gtc cta cgg ttt gac aac acc 1104
Leu Thr Cys Ser Glu Pro Gly Ile Tyr Val Leu Arg Phe Asp Asn Thr
        355                 360                 365

tac agc ttc atc cat gcc aag aaa gtc agt ttc act gtg gag gtc ctg 1152
Tyr Ser Phe Ile His Ala Lys Lys Val Ser Phe Thr Val Glu Val Leu
    370                 375                 380

ctt cca gac aaa gca gca gaa gag aag ttg aat cag cag ggg gca gtc 1200
Leu Pro Asp Lys Ala Ala Glu Glu Lys Leu Asn Gln Gln Gly Ala Val
385                 390                 395                 400

acc ccc aaa taa 1212
Thr Pro Lys

<210> 2
<211> 403
<212> PRT
<213> Rattus norvegicus

<400> 2
Met Ser Gly Arg Val Gly Asp Leu Ser Pro Lys Gln Glu Glu Ala Leu
1               5                   10                  15

Ala Lys Phe Arg Glu Asn Val Gln Asp Val Leu Pro Ala Leu Pro Asn
            20                  25                  30

Pro Asp Asp Tyr Phe Leu Leu Arg Trp Leu Arg Ala Arg Ser Phe Asp
        35                  40                  45

Leu Gln Lys Ser Glu Ala Met Leu Arg Lys His Val Glu Phe Arg Lys

50                  55                  60

Gln Lys Asp Ile Asp Lys Ile Ile Ser Trp Gln Pro Pro Glu Val Ile
65                  70                  75                  80

Gln Gln Tyr Leu Ser Gly Gly Arg Cys Gly Tyr Asp Leu Asp Gly Cys
                85                  90                  95

Pro Val Trp Tyr Asp Ile Ile Gly Pro Leu Asp Ala Lys Gly Leu Leu
            100                 105                 110

Phe Ser Ala Ser Lys Gln Asp Leu Leu Arg Thr Lys Met Arg Asp Cys
        115                 120                 125

Glu Leu Leu Leu Gln Glu Cys Thr Gln Gln Thr Ala Lys Leu Gly Lys
    130                 135                 140

Lys Ile Glu Thr Ile Thr Met Ile Tyr Asp Cys Glu Gly Leu Gly Leu
145                 150                 155                 160

Lys His Leu Trp Lys Pro Ala Val Glu Ala Tyr Gly Glu Phe Leu Thr
                165                 170                 175

Met Phe Glu Glu Asn Tyr Pro Glu Thr Leu Lys Arg Leu Phe Val Val
            180                 185                 190

Lys Ala Pro Lys Leu Phe Pro Val Ala Tyr Asn Leu Ile Lys Pro Phe
        195                 200                 205

Leu Ser Glu Asp Thr Arg Lys Lys Ile Met Val Leu Gly Ala Asn Trp
    210                 215                 220

Lys Glu Val Leu Leu Lys His Ile Ser Pro Asp Gln Leu Pro Val Glu
225                 230                 235                 240

Tyr Gly Gly Thr Met Thr Asp Pro Asp Gly Asn Pro Lys Cys Lys Ser
                245                 250                 255

Lys Ile Asn Tyr Gly Gly Asp Ile Pro Lys Gln Tyr Tyr Val Arg Asp
            260                 265                 270

Gln Val Lys Gln Gln Tyr Glu His Ser Val Gln Ile Ser Arg Gly Ser
        275                 280                 285

Ser His Gln Val Glu Tyr Glu Ile Leu Phe Pro Gly Cys Val Leu Arg
    290                 295                 300

Trp Gln Phe Met Ser Glu Gly Ser Asp Val Gly Phe Gly Ile Phe Leu
305                 310                 315                 320

Lys Thr Lys Met Gly Glu Arg Gln Arg Ala Gly Glu Met Thr Glu Val
                325                 330                 335

Leu Pro Asn Gln Arg Tyr Asn Ser His Met Val Pro Glu Asp Gly Thr
            340                 345                 350

Leu Thr Cys Ser Glu Pro Gly Ile Tyr Val Leu Arg Phe Asp Asn Thr
        355                 360                 365

Tyr Ser Phe Ile His Ala Lys Lys Val Ser Phe Thr Val Glu Val Leu
    370                 375                 380

Leu Pro Asp Lys Ala Ala Glu Glu Lys Leu Asn Gln Gln Gly Ala Val
385                 390                 395                 400

Thr Pro Lys

<210> 3
<211> 1212
<212> DNA
<213> Homo sapience

<220>
<221> CDS
<222> (1)..(1209)

<400> 3
atg agc ggc aga gtc ggc gat ctg agc ccc agg cag aag gag gca ttg 48
Met Ser Gly Arg Val Gly Asp Leu Ser Pro Arg Gln Lys Glu Ala Leu
1               5                   10                  15

gcc aag ttt cgg gag aat gtc cag gat gtg ctg ccg gcc ctg ccg aat 96
Ala Lys Phe Arg Glu Asn Val Gln Asp Val Leu Pro Ala Leu Pro Asn
            20                  25                  30

cca gat gac tat ttt ctc ctg cgt tgg ctc cga gcc aga agc ttc gac 144
Pro Asp Asp Tyr Phe Leu Leu Arg Trp Leu Arg Ala Arg Ser Phe Asp
        35                  40                  45

ctg cag aag tcg gag gcc atg ctc cgg aag cat gtg gag ttc cga aag 192
Leu Gln Lys Ser Glu Ala Met Leu Arg Lys His Val Glu Phe Arg Lys
    50                  55                  60

caa aag gac att gac aac atc att agc tgg cag cct cca gag gtg atc 240
Gln Lys Asp Ile Asp Asn Ile Ile Ser Trp Gln Pro Pro Glu Val Ile
65                  70                  75                  80

caa cag tat ctg tca ggg ggt atg tgt ggc tat gac ctg gat ggc tgc 288
Gln Gln Tyr Leu Ser Gly Gly Met Cys Gly Tyr Asp Leu Asp Gly Cys
                85                  90                  95

cca gtc tgg tac gac ata att gga cct ctg gat gcc aag ggt ctg ctg 336
Pro Val Trp Tyr Asp Ile Ile Gly Pro Leu Asp Ala Lys Gly Leu Leu
            100                 105                 110

ttc tca gcc tcc aaa cag gac ctg ctg agg acc aag atg cgg gag tgt 384
Phe Ser Ala Ser Lys Gln Asp Leu Leu Arg Thr Lys Met Arg Glu Cys
        115                 120                 125

gag ctg ctt ctg caa gag tgt gcc cac cag acc aca aag ttg ggg agg 432
Glu Leu Leu Leu Gln Glu Cys Ala His Gln Thr Thr Lys Leu Gly Arg
    130                 135                 140

aag gtg gag acc atc acc ata att tat gac tgc gag ggg ctt ggc ctc 480

Lys Val Glu Thr Ile Thr Ile Ile Tyr Asp Cys Glu Gly Leu Gly Leu
145                 150                 155                 160

aag cat ctc tgg aag cct gct gtg gag gcc tat gga gag ttt ctc tgc     528
Lys His Leu Trp Lys Pro Ala Val Glu Ala Tyr Gly Glu Phe Leu Cys
                165                 170                 175

atg ttt gag gaa aat tat ccc gaa aca ctg aag cgt ctt ttt gtt gtt     576
Met Phe Glu Glu Asn Tyr Pro Glu Thr Leu Lys Arg Leu Phe Val Val
            180                 185                 190

aaa gcc ccc aaa ctg ttt cct gtg gcc tat aac ctc atc aaa ccc ttc     624
Lys Ala Pro Lys Leu Phe Pro Val Ala Tyr Asn Leu Ile Lys Pro Phe
        195                 200                 205

ctg agt gag gac act cgt aag aag atc atg gtc ctg gga gca aat tgg     672
Leu Ser Glu Asp Thr Arg Lys Lys Ile Met Val Leu Gly Ala Asn Trp
    210                 215                 220

aag gag gtt tta ctg aaa cat atc agc cct gac cag gtg cct gtg gag     720
Lys Glu Val Leu Leu Lys His Ile Ser Pro Asp Gln Val Pro Val Glu
225                 230                 235                 240

tat ggg ggc acc atg act gac cct gat gga aac ccc aag tgc aaa tcc     768
Tyr Gly Gly Thr Met Thr Asp Pro Asp Gly Asn Pro Lys Cys Lys Ser
                245                 250                 255

aag atc aac tac ggg ggt gac atc ccc agg aag tat tat gtg cga gac     816
Lys Ile Asn Tyr Gly Gly Asp Ile Pro Arg Lys Tyr Tyr Val Arg Asp

            260                 265                 270

cag gtg aaa cag cag tat gaa cac agc gtg cag att tcc cgt ggc tcc 864
Gln Val Lys Gln Gln Tyr Glu His Ser Val Gln Ile Ser Arg Gly Ser
        275                 280                 285

tcc cac caa gtg gag tat gag atc ctc ttc cct ggc tgt gtc ctc agg 912
Ser His Gln Val Glu Tyr Glu Ile Leu Phe Pro Gly Cys Val Leu Arg
    290                 295                 300

tgg cag ttt atg tca gat gga gcg gat gtt ggt ttt ggg att ttc ctg 960
Trp Gln Phe Met Ser Asp Gly Ala Asp Val Gly Phe Gly Ile Phe Leu
305                 310                 315                 320

aag acc aag atg gga gag agg cag cgg gca ggg gag atg aca gag gtg 1008
Lys Thr Lys Met Gly Glu Arg Gln Arg Ala Gly Glu Met Thr Glu Val
                325                 330                 335

ctg ccc aac cag agg tac aac tcc cac ctg gtc cct gaa gat ggg acc 1056
Leu Pro Asn Gln Arg Tyr Asn Ser His Leu Val Pro Glu Asp Gly Thr
            340                 345                 350

ctc acc tgc agt gat cct ggc atc tat gtc ctg cgg ttt gac aac acc 1104
Leu Thr Cys Ser Asp Pro Gly Ile Tyr Val Leu Arg Phe Asp Asn Thr
        355                 360                 365

tac agc ttc att cat gcc aag aag gtc aat ttc act gtg gag gtc ctg 1152
Tyr Ser Phe Ile His Ala Lys Lys Val Asn Phe Thr Val Glu Val Leu
    370                 375                 380

ctt cca gac aaa gcc tca gaa gag aag atg aaa cag ctg ggg gca ggc 1200
Leu Pro Asp Lys Ala Ser Glu Glu Lys Met Lys Gln Leu Gly Ala Gly
385                 390                 395                 400

acc ccg aaa taa 1212
Thr Pro Lys

<210> 4
<211> 403
<212> PRT
<213> Homo sapience

<400> 4
Met Ser Gly Arg Val Gly Asp Leu Ser Pro Arg Gln Lys Glu Ala Leu
1               5                   10                  15

Ala Lys Phe Arg Glu Asn Val Gln Asp Val Leu Pro Ala Leu Pro Asn
            20                  25                  30

Pro Asp Asp Tyr Phe Leu Leu Arg Trp Leu Arg Ala Arg Ser Phe Asp
        35                  40                  45

Leu Gln Lys Ser Glu Ala Met Leu Arg Lys His Val Glu Phe Arg Lys
    50                  55                  60

Gln Lys Asp Ile Asp Asn Ile Ile Ser Trp Gln Pro Pro Glu Val Ile
65                  70                  75                  80

Gln Gln Tyr Leu Ser Gly Gly Met Cys Gly Tyr Asp Leu Asp Gly Cys
                85                  90                  95

Pro Val Trp Tyr Asp Ile Ile Gly Pro Leu Asp Ala Lys Gly Leu Leu
            100                 105                 110

Phe Ser Ala Ser Lys Gln Asp Leu Leu Arg Thr Lys Met Arg Glu Cys
        115                 120                 125

Glu Leu Leu Leu Gln Glu Cys Ala His Gln Thr Thr Lys Leu Gly Arg
    130                 135                 140

Lys Val Glu Thr Ile Thr Ile Ile Tyr Asp Cys Glu Gly Leu Gly Leu
145                 150                 155                 160

Lys His Leu Trp Lys Pro Ala Val Glu Ala Tyr Gly Glu Phe Leu Cys
                165                 170                 175

Met Phe Glu Glu Asn Tyr Pro Glu Thr Leu Lys Arg Leu Phe Val Val
            180                 185                 190

Lys Ala Pro Lys Leu Phe Pro Val Ala Tyr Asn Leu Ile Lys Pro Phe
        195                 200                 205

Leu Ser Glu Asp Thr Arg Lys Lys Ile Met Val Leu Gly Ala Asn Trp
    210                 215                 220

Lys Glu Val Leu Leu Lys His Ile Ser Pro Asp Gln Val Pro Val Glu

225                 230                 235                 240

Tyr Gly Gly Thr Met Thr Asp Pro Asp Gly Asn Pro Lys Cys Lys Ser
                245                 250                 255

Lys Ile Asn Tyr Gly Gly Asp Ile Pro Arg Lys Tyr Tyr Val Arg Asp
            260                 265                 270

Gln Val Lys Gln Gln Tyr Glu His Ser Val Gln Ile Ser Arg Gly Ser
        275                 280                 285

Ser His Gln Val Glu Tyr Glu Ile Leu Phe Pro Gly Cys Val Leu Arg
    290                 295                 300

Trp Gln Phe Met Ser Asp Gly Ala Asp Val Gly Phe Gly Ile Phe Leu
305                 310                 315                 320

Lys Thr Lys Met Gly Glu Arg Gln Arg Ala Gly Glu Met Thr Glu Val
                325                 330                 335

Leu Pro Asn Gln Arg Tyr Asn Ser His Leu Val Pro Glu Asp Gly Thr
            340                 345                 350

Leu Thr Cys Ser Asp Pro Gly Ile Tyr Val Leu Arg Phe Asp Asn Thr
        355                 360                 365

Tyr Ser Phe Ile His Ala Lys Lys Val Asn Phe Thr Val Glu Val Leu
    370                 375                 380

Leu Pro Asp Lys Ala Ser Glu Glu Lys Met Lys Gln Leu Gly Ala Gly
385                 390                 395                 400

Thr Pro Lys

<210> 5
<211> 19
<212> PRT
<213> Rattus norvegicus

<400> 5
His Ile Ser Pro Asp Gln Leu Pro Val Glu Tyr Gly Gly Thr Met Thr
  1               5                  10                  15
Asp Pro Asp

<210> 6
<211> 19
<212> PRT
<213> Rattus norvegicus

<400> 6
Glu Asn Val Gln Asp Val Leu Pro Ala Leu Pro Asn Pro Asp Asp Tyr
  1               5                  10                  15
Phe Leu Leu

<210> 7

<211> 20
<212> DNA
<213> Artficial Sequence

<400> 7
cagcgcgccc caaggctgag                                                    20

<210> 8
<211> 20
<212> DNA
<213> Artficial Sequence

<400> 8
gactaaagaa actgacagga                                                    20

<210> 9
<211> 20
<212> DNA
<213> Artficial Sequence

<400> 9
atgagcggca gagtcggcga                                                    20

<210> 10
<211> 20
<212> DNA
<213> Artficial Sequence

<400> 10

ttatttcggg gtgcctgccc 20

<210> 11
<211> 32
<212> DNA
<213> Artficial Sequence

<400> 11
atggatccat gagcggcaga gtcggtgacc tg 32

<210> 12
<211> 26
<212> DNA
<213> Artficial Sequence

<400> 12
aagtcgactt atttgggggt gtctgc 26

<210> 13
<211> 28
<212> DNA
<213> Artficial Sequence

<400> 13
ccggaattca tgagcggcag agtcggcg 28

<210> 14
<211> 27
<212> DNA

<213> Artficial Sequence

<400> 14
cgcgtcgact tatttcgggg tgcctgc 27

<210> 15
<211> 21
<212> DNA
<213> Artficial Sequence

<400> 15
ttctcgagtt tgggggtgac t 21

<210> 16
<211> 21
<212> DNA
<213> Artficial Sequence

<400> 16
tactcgagtt tcggggtgcc t 21